# EUROPÄISCHE PATENTSCHRIFT

(11) **EP 1 575 559 B1**
(45) Veröffentlichungstag und Bekanntmachung des Hinweises auf die Patenterteilung: **09.08.2006**
(21) Anmeldenummer: 03799507.3
(22) Anmeldetag: 24.12.2003
(51) Int. Cl.: A61K 9/12

(54) **DOSIERAEROSOLE MIT LECITHIN ALS OBERFLÄCHENAKTIVER SUBSTANZ**
DOSING AEROSOLS CONTAINING LECITHIN AS A SURFACE-ACTIVE SUBSTANCE
AEROSOLS-DOSEURS CONTENANT DE LA LECITHINE COMME SURFACTANT

(30) Priorität: 24.12.2002 DE 10260882
(43) Veröffentlichungstag der Anmeldung: 21.09.2005
(73) Patentinhaber: IG Sprühtechnik GmbH & Co. Kg, 79664 Wehr (DE)
(72) Erfinder: GUCK, Franz, 79618 Rheinfelden (DE); WARNKE, Gieselher, 79737 Herrischried (DE)
(74) Vertreter: Flaccus, Rolf-Dieter
(86) Internationale Anmeldenummer: PCT/EP2003/014901
(87) Internationale Veröffentlichungsnummer: WO 2004/058221

(56) Entgegenhaltungen:
- WO-A-93/04671
- DE-A- 19 911 064

## Beschreibung

Gegenstand der vorliegenden Erfindung sind Dosieraerosole, die mindestens einen Arzneistoff sowie ein Gemisch aus druckverflüssigtem Isobutan als Treibmittel und Lecithin als oberflächenaktiver Substanz umfassen.

Die vorliegende Erfindung betrifft insbesondere Dosieraerosole, die mindestens einen antiasthmatisch wirksamen Arzneistoff aus der Gruppe der Glucocorticoide sowie ein Gemisch aus druckverflüssigtem Isobutan als Treibmittel und Lecithin umfassen.

Aerosol-Druckgaspackungen, kurz Dosieraerosole genannt, die unter Verwendung druckverflüssigter oder komprimierter Gase als Treibmittel hergestellt und verwendet werden, sind seit langem bekannt. Solche Dosieraerosole umfassen im allgemeinen einen Druckbehälter, vorzugsweise aus Metall oder Glas mit einer Ventilkonstruktion zur Entnahme des Inhalts und dem eigentlichen, zu versprühenden Mittel, das in den meisten Fällen aus einer Wirkstofflösung sowie einem Treibmittel in Form eines druckverflüssigten Gases oder Gasgemisches besteht. Das druckverflüssigte Gas bzw. die druckverflüssigten Gasgemische sollten idealerweise in jedem Verhältnis mit dem Wirkstoff mischbar sein, so dass eine einzige flüssige Phase entsteht. Alternativ dazu sollte das druckverflüssigte Gas bzw. Gasgemisch eine gut aufzuschüttelnde Suspension bilden, über der sich eine Gasphase bildet. Je nach enthaltenem Wirkstoff werden die Dosieraerosole im kosmetischen oder medizinischen Bereich, aber auch als Raumspray, Insektizidspray oder ähnliches eingesetzt.

Die Treibmittel von Dosieraerosolen haben besondere Anforderungen zu erfüllen. Sie dürfen mit den Bestandteilen der Wirkstofflösung keine Reaktion eingehen. Die Treibmittel dürfen auch nicht irritierend und nicht toxisch sein. Als besonders gut geeignete Treibmittel hatten sich Fluorchlorkohlenwasserstoffe erwiesen. Aufgrund ihrer Ozon abbauenden Wirkung war es jedoch notwendig, alternative Treibmittel zu entwickeln. Die alternativen Treibmittel müssen jedoch qualitativ den Fluorchlorkohlenwasserstoffen entsprechen, d. h. sie müssen vor allem gesundheitlich unbedenklich und zudem ökologisch verträglich sein. Zunächst wurden teilhalogenierte Fluorchlorkohlenwasserstoffe als Ersatztreibmittel propagiert, jedoch weisen auch sie noch immer eine inakzeptabel hohe Ozon abbauende Wirkung auf.

In der DE 41 32 176 werden Dosieraerosole zur Verabreichung von Isoprenalin-Abkömmlingen, den so genannten β-Sympathomimetika, oder dem nichtsteroidalen Entzündungshemmer DNCG offenbart, bei denen Isobutan als Treibmittel verwendet wird.

In der DE 199 11 064 werden Dosieraerosole mit broncholytischen und/oder entzündungshemmenden Wirkstoffen aus der Gruppe der Glucocorticoide mit Isobutan als Treibmittel und Ölsäure oder Span 85 als oberflächenaktive Substanzen beschrieben. Diese Dosieraerosole weisen jedoch die Nachteile einer nicht zufrieden stellenden Resuspendierbarkeit und zu schnellen Sedimentation des Wirkstoffs in dem Treibmittel auf.

Es war daher Aufgabe der vorliegenden Erfindung, ein Dosieraerosol für Arzneistoffe, insbesondere für antiasthmatisch wirksame Arzneistoffe aus der Gruppe der Glucocorticoide bereitzustellen, das die Nachteile der aus DE 199 11 064 bekannten Dosieraerosole nicht aufweist.

Überraschenderweise wurde festgestellt, dass der Hilfsstoff Lecithin zu einer deutlichen Verbesserung der Resuspendierbarkeit von Arzneistoffen, insbesondere von Glucocorticoiden in Isobutan führt.

Lecithine sind Glycero-Phospholipide, die aus Fettsäuren, Glycerol, Phosphorsäure und Cholin gebildet werden. Natürlich vorkommende Lecithine sind Derivate der 1,2-Diacyl-sn-glycerol-3-Phosphorsäure. Bei Extraktion aus biologischem Material wird immer eine Mischung von Lecithinen erhalten, die sich durch die unterschiedlichen Fettsäurereste voneinander unterscheiden.

Das erfindungsgemäß bevorzugte Lecithin ist Sojalecithin, das als Emulgator in der pharmazeutischen Industrie bereits vielfach eingesetzt wird.

Beim Vergleich des Sedimentationsverhaltens von Arzneistoffsuspensionen in Isobutan unter Zusatz von Sojalecithin oder verschiedenen, üblicherweise für die Herstellung von antiasthmatischen Dosieraerosolen eingesetzten oberflächenaktiven Substanzen wurde, wie aus dem nachfolgenden Beispiel ersichtlich wird, beobachtet, dass die Arzneistoffsuspension mit Sojalecithin 10 mal solange zur Sedimentation benötigte wie eine Arzneistoffsuspension mit Ölsäure und 5 mal solange wie eine Arzneistoffsuspension mit Span 85.

Bei weiteren Versuchen, in denen das Verhältnis von Arzneistoff zu Sojalecithin 1:2, 1:1 oder 1:0,5 betrug, wurden keine Unterschiede in den Sedimentationszeiten beobachtet, so dass vorteilhafterweise ein Verhältnis von Arzneistoff zu Sojalecithin von 1:0,5 gewählt werden kann.

### Beispiel

Vergleich des Suspensionsverhaltens von Arzneistoffsuspensionen in Isobutan bei Verwendung verschiedener oberflächenaktiver Substanzen.

| | Relative Sedimentation |
|---|---|
| Glucocorticoid : Ölsäure (100:1) | 1 |
| Glucocorticoid : Span 85 (1:1) | 2 |
| Glucocorticoid : Sojalecithin (1:2) | 10 |
| Glucocorticoid : Sojalecithin (1:1) | 10 |
| Glucocorticoid : Sojalecithin (1:0,5) | 10 |

In weiteren Versuchen haben sich die nachfolgenden Rezepturbilder als besonders vorteilhaft herausgestellt:

| | | |
|---|---|---|
| Rezepturbild 1: | Glucocorticoid | 0,1% - 0,2% |
| | Lecithin | 0,05% - 0,4% |
| | Isobutan | 99,85% - 99,4% |

| | | |
|---|---|---|
| Rezepturbild 2: | Glucocorticoid | 0,5% - 1,0% |
| | Lecithin | 0,25% - 4,0% |
| | Isobutan | 99,25% - 95,0% |

Für das Glucocorticoid Beclomethasondiproprionat wurde das folgende Rezepturbild als günstig gefunden:

| | | |
|---|---|---|
| Rezepturbild 3: | Beclomethason | 0,1% - 2,5% |
| | Sojalecithin | 0,05% - 5,0% |
| | Isobutan | 99,85% - 92,5% |

Für Budesonid erwies sich das folgende Rezepturbild als äußerst günstig:

| | | |
|---|---|---|
| Rezepturbild 4: | Budesonid | 0,1% - 2,5% |
| | Sojalecithin | 0,05% - 5,0% |
| | Isobutan | 99,85% - 92,5% |

Alle Mengenangaben beziehen sich auf Gew.-%.

Die erfindungsgemäßen Aerosole können durch Mischen der verschiedenen Bestandteile unter Bedingungen, bei denen das Treibmittel und das oberflächenaktive Mittel flüssig sind und der Wirkstoff in fester Phase vorliegt, hergestellt werden.

Die Arzneistoffsuspension wird durch das Ventil unter Druck in die verclinchte Dose, die zu Beginn des Füllvorgangs Raumtempqratur hat, gefüllt. Die Suspension hat eine Temperatur von ca. -10 bis +10°C. Anschließend wird mit dem Treibmittel nachgefüllt und so das Ventil gleichzeitig gereinigt.

Die erfindungsgemäßen Dosieraerosole können bei der Behandlung von Mensch und Tier, insbesondere bei der Behandlung allergischer Erkrankungen der Luftwege wie Asthma oder allergischer Rhinitis (Heuschnupfen), vorzugsweise durch orale oder nasale Inhalation eingesetzt werden.

## Patentansprüche

1. Dosieraerosol mit zumindest einem Arzneistoff, einem Treibmittel und Lecithin als oberflächenaktiver Substanz, **dadurch gekennzeichnet, dass** der Arzneistoff in Suspension vorliegt und das Treibmittel druckverflüssigtes Isobutan ist.

2. Dosieraerosol nach Anspruch 1, **dadurch gekennzeichnet, dass** der Arzneistoff ein Qlucocorticoid ist, wobei das Glucocorticoid vorzugsweise aus der Gruppe ausgewählt ist, die aus Cortisol, Prednison, Prednisolon, Methylprednisolon, Triamionolon, Prednyliden, Fluocortolon, Paramethason, Dexamethason, Betamethason, Flunisolid, Fluticason, Baclometason, Budesonid und/oder deren antiasthmatisch wirksamen Derivaten und/oder deren Mischungen besteht.

3. Dosieraerosol nach Anspruch 1 oder 2, **dadurch gekennzeichnet, dass** es dem Rerepturbild
| | |
|---|---|
| Glucocorticoid | 0,1% - 0,2% |
| Lecithin | 0,05% - 0,4% |
| Isobutan | 99,85% - 99,4% |
entspricht.

4. Dosieraurosol nach Anspruch 1 oder 2, **dadurch gekennzeichnet, dass** es dem Rerepturbild
| | |
|---|---|
| Qlucocorticoid | 0,5% - 1,0% |
| Lecithin | 0,25% - 4,0% |
| Isobutan | 99,25% - 95,0% |
entspricht.

5. Dosieraerosol nach einem der vorangehanden Ansprüche, **dadurch gekennzeichnet, dass** das Lecithin Sojalecithin ist.

6. Dosieraerosol nach Anspruch 1 oder 2, **dadurch gekennzeichnet, dass** es dem Rezepturbild
| | |
|---|---|
| Beclomethason | 0,1% - 2,5% |
| Sojalecithin | 0,05% - 5,0% |
| Isobutan | 99,85% - 92,5% |
entspricht.

7. Dosieraerosol nach Anspruch 1 oder 2, **dadurch gekennzeichnet, dass** es dem Rezepturbild
| | |
|---|---|
| Budesonid | 0,1% - 2,5% |
| Sojalecithin | 0,05% - 5,0% |
| Isobutan | 99,85% - 92,5% |
entspricht.

8. Dosieraerosol nach einem der vorangehenden Ansprüche, **dadurch gekennzeichnet, dass** das Verhältnis von Clucocorticoid zu Sojalecithin 1:2, vorzugsweise 1:1 und besonders bevorzugt 1:0,5 beträgt.

9. Dosieraerosol nach einem der vorangehenden Ansprüche, zur Behandlung allergischer Erkrankungen bei Mensch und Tier, vorzugsweise zur Inhalationsbehandlung allergischer Erkrankungen der Luftwege.

10. Dosieraerosol nach einem der Ansprüche 1 bis 8 zur Behandlung von Asthma oder allergischer Rhinitis.

11. Verfahren zur Herstellung von Dosieraerosolen nach einem der vorangehenden Ansprüche, **dadurch gekennzeichnet, dass** Isobutan als Treibmittel in flüssiger Form und Lecithin als oberflächenaktives Mittel in flüssiger Form und zumindest ein Arzneistoff als Feststoff miteinander vermischt werden, und die flüssige Suspension unter Druck in die vorgesehene Sprühdose eingefüllt wird.

12. Verfahren nach Anspruch 11, **dadurch gekennzeichnet, daß** die Temperatur nach dem Einfüllen der Suspension zwischen -10 und +10°C liegt.

13. Verfahren nach Anspruch 11 oder 12, **dadurch gekennzeichnet, daß** das Ventil der Sprühdose unter Nachfüllen von Treibmittel nach dem Einfüllen der Suspension gereinigt wird.

## Claims

1. Controlled dosage aerosol with at least one medicinal agent, a propellant, and lecithin as surface-active agent, **characterized in that** the said medicinal agent is present in the form of a suspension and that the said propellant is pressure-liquefied isobutane.

2. Controlled dosage aerosol according to claim 1, **characterized in that** the said medicinal agent is a glucocorticoid, said glucocorticoid preferably being selected from the group consisting of cortisol, prednisone, prednisolone, methylprednisolone, triamcinolone, prednylidene, fluocortolone, paramethasone, dexamethasone, betamethasone, flunisolide, fluticasone, beclomethasone, budesonide and/or their anti-asthmatically active derivatives and/or mixtures thereof.

3. Controlled dosage aerosol according to claim 1 or 2, **characterized in that** it corresponds to the formulation
| | |
|---|---|
| Glucocorticoid | 0.1% - 0.2% |
| Lecithin | 0.05% - 0.4% |
| Isobutane | 99.85% - 99.4% |

4. Controlled dosage aerosol according to claim 1 or 2, **characterized in that** it corresponds to the formulation
| | |
|---|---|
| Glucocorticoid | 0.5% - 1.0% |
| Lecithin | 0.25% - 4.0% |
| Isobutane | 99.25% - 95.0% |

5. Controlled dosage aerosol according to any one of the preceding claims, **characterized in that** the said lecithin is soybean lecithin.

6. Controlled dosage aerosol according to claim 1 or 2, **characterized in that** it corresponds to the formulation
| | |
|---|---|
| Beclomethasone | 0.1% - 2.5% |
| Soybean lecithin | 0.05% - 5.0% |
| Isobutane | 99.85% - 92.5% |

7. Controlled dosage aerosol according to claim 1 or 2, **characterized in that** it corresponds to the formulation
| | |
|---|---|
| Budesonide | 0.1% - 2.5% |
| Soybean lecithin | 0.05% - 5.0% |
| Isobutane | 99.85% - 92.5% |

8. Controlled dosage aerosol according to any one of the preceding claims, **characterized in that** the ratio of glucocorticoid and soybean lecithin is 1:2, preferably 1:1, and with particular preference 1:0.5.

9. Controlled dosage aerosol according to any one of the preceding claims for treating allergic diseases in humans and animals, preferably for inhalation treatment of allergic diseases of the respiratory tract.

10. Controlled dosage aerosol according to any one of claims 1 to 8, for treating asthma or allergic rhinitis.

11. Process for the production of controlled dosage aerosols according to any one of the preceding claims, **characterized in that** isobutane as a propellant in liquid form, and lecithin as a surface-active agent in liquid form, and at least one medicinal agent as solid substance are mixed with one other, and that the liquid suspension is filled under pressure into the spray tin provided therefore.

12. Process according to claim 11, **characterized in that** after filling in the suspension the temperature is between -10 and +10°C.

13. Process according to claim 11 or 12, **characterized in that** after filling in the suspension, the valve of the spray tin is cleaned by filling the tin up with propellant.

## Revendications

1. Aérosol-doseur comprenant au moins un médicament, un agent propulseur et de la lécithine comme substance tensioactive, **caractérisé en ce que** le médicament se trouve en suspension et l'agent propulseur est de l'isobutane liquéfié sous pression.

2. Aérosol-doseur selon la revendication 1, **caractérisé en ce que** le médicament est un glucocorticoïde, le glucocorticoïde étant de préférence choisi dans le groupe constitué par le cortisol, la prednisone, la prednisolone, la méthylprednisolone, la triamionolone, la prednylidone, la fluocortolone, la paraméthasone, la dexaméthasone, la bétaméthasone, le flunisolide, la fluticasone, la béclométasone, le budésonide et/ou leurs dérivés à activité antiasthmatique et/ou leurs mélanges.

3. Aérosol-doseur selon la revendication 1 ou 2, **caractérisé en ce qu'**il correspond à la formulation
| | |
|---|---|
| Glucocorticoïde | 0,1% - 0,2% |
| Lécithine | 0,05% - 0,4% |
| Isobutane | 99,85% - 99,4%. |

4. Aérosol-doseur selon la revendication 1 ou 2, **caractérisé en ce qu'**il correspond à la formulation
| | |
|---|---|
| Glucocorticoïde | 0,5% - 1,0% |
| Lécithine | 0,25% - 4,0% |
| Isobutane | 99,25% - 95,0%. |

5. Aérosol-doseur selon l'une quelconque des revendications précédentes, **caractérisé en ce que** la lécithine est la lécithine de soja.

6. Aérosol-doseur selon la revendication 1 ou 2, **caractérisé en ce qu'**il correspond à la formulation
| | |
|---|---|
| Béclométhasone | 0,1% - 2,5% |
| Lécithine de soja | 0,05% - 5,0% |
| Isobutane | 99,85% - 92,5%. |

7. Aérosol-doseur selon la revendication 1 ou 2, **caractérisé en ce qu'**il correspond à la formulation
| | |
|---|---|
| Budésonide | 0,1% - 2,5% |
| Lécithine de soja | 0,05% - 5,0% |
| Isobutane | 99,85% - 92,5%. |

8. Aérosol-doseur selon l'une quelconque des revendications précédentes, **caractérisé en ce que** le rapport glucocorticoïde à lécithine de soja est 1:2, de préférence 1:1 et de manière particulièrement préférée 1:0,5.

9. Aérosol-doseur selon l'une quelconque des revendications précédentes, pour le traitement de maladies allergiques chez l'homme et l'animal, de préférence pour le traitement par inhalation de maladies allergiques des voies respiratoires.

10. Aérosol-doseur selon l'une quelconque des revendications 1 à 8 pour le traitement de l'asthme ou de la rhinite allergique.

11. Procédé pour la préparation d'aérosols-doseurs selon l'une quelconque des revendications précédentes, **caractérisé en ce qu'**on mélange l'isobutane comme agent propulseur sous forme liquide et la lécithine comme agent tensioactif sous forme liquide et au moins un médicament comme solide les uns avec les autres et on transvase la suspension liquide sous pression dans le pulvérisateur prévu.

12. Procédé selon la revendication 11, **caractérisé en ce que** la température après le transvasement de la suspension est comprise entre -10 et +10°C.

13. Procédé selon la revendication 11 ou 12, **caractérisé en ce que** la soupape du pulvérisateur est nettoyée après le transvasement de la suspension par un remplissage ultérieur d'agent propulseur.
